(19) Europäisches Patentamt
European Patent Office
Office européen des brevets

(11) Veröffentlichungsnummer: **0 130 142**
**B1**

(12) # EUROPÄISCHE PATENTSCHRIFT

(45) Veröffentlichungstag der Patentschrift: **31.05.89**

(21) Anmeldenummer: **84730071.2**

(22) Anmeldetag: **22.06.84**

(51) Int. Cl.⁴: **C 07 D 307/935,** C 07 D 407/06, A 61 K 31/557, A 61 K 31/335 // C07D317/06, C07D317/30, C07D319/06, C07C47/14, C07C43/303

(54) **Neue Prostacyclinderivate, Verfahren zu ihrer Herstellung und ihre Verwendung als Arzneimittel.**

(30) Priorität: **23.06.83 DE 3322893**

(43) Veröffentlichungstag der Anmeldung: **02.01.85 Patentblatt 85/1**

(45) Bekanntmachung des Hinweises auf die Patenterteilung: **31.05.89 Patentblatt 89/22**

(84) Benannte Vertragsstaaten: **AT BE CH DE FR GB IT LI LU NL SE**

(56) Entgegenhaltungen:
**EP-A-0 002 234**
**EP-A-0 051 558**
**DE-A-2 500 451**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Dahl, Helmut, Dr., Gollanczstrasse 102, D-1000 Berlin 28 (DE)**
Erfinder: **Radüchel, Bernd, Dr., Gollanczstrasse 132, D-1000 Berlin 28 (DE)**
Erfinder: **Vorbrüggen, Helmut, Prof., Wilkestrasse 7, D-1000 Berlin 27 (DE)**
Erfinder: **Loge, Olaf, Dr., Bekassinenweg 37, D-1000 Berlin 27 (DE)**

EP 0 130 142 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue Prostacyclin-Derivate, Verfahren zu ihrer Herstellung sowie ihre Verwendung als Arzneimittel.

Aus dem sehr umfangreichen Stand der Technik der Prostacycline und deren Analoga weiß man, daß diese Stoffklasse aufgrund ihrer biologischen und pharmakologischen Eigenschaften zur Behandlung von Säugetieren, einschließlich Menschen geeignet ist. Ihre Verwendung stößt aber auf Schwierigkeiten, da sie chemisch instabil sind und für therapeutische Zwecke eine zu kurze Wirkungsdauer besitzen. Alle Strukturveränderungen haben das Ziel, die Wirkungsdauer sowie die Selektivität der Wirksamkeit zu steigern.

In der DE-OS-2 753 244 werden Prostacyclinderivate, die durch eine Nitrilgruppe an der Enolätherdoppelbindung stabilisiert sind, beschrieben.

Es wurde nun gefunden, daß durch Ersatz der 1-Carboxylgruppe in den 5-Cyano-prostacyclinen durch eine Acetalgruppe eine längere Wirkungsdauer und größere Selektivität erzielt werden kann.

Die erfindungsgemäßen Verbindungen sind besonders zur Inhibierung der Magensäuresekretion und zur Cytoprotektion am Magen, Herzen und an der Leber geeignet.

Die Erfindung betrifft 5-Cyanoprostacycline der allgemeinen Formel I

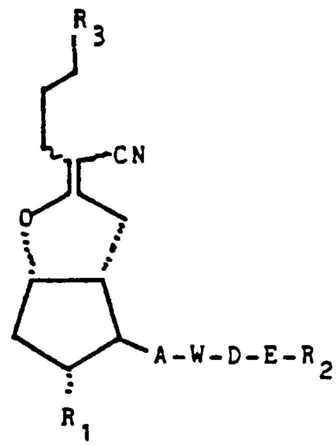

worin

A eine trans-CH = CH- oder -C≡C-Gruppe,

W eine Hydroxymethylen-, Acetoxymethylen-,

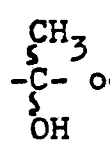 oder 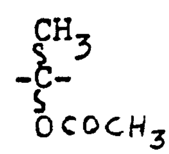

Gruppe,

wobei

die OH- oder Acetoxy-Gruppe α- oder β-ständig sein kann,

D und E gemeinsam eine direkte Bindung oder

D eine geradkettige Alkylengruppe mit 1 bis 5 C-Atomen oder eine verzweigte Alkylengruppe mit 2 bis 5 C-Atomen,

E ein Sauerstoffatom, eine -C≡C-Bindung oder eine direkte Bindung,

$R_2$ eine $C_1$-$C_7$-Alkyl-Gruppe oder eine Phenyl-Gruppe,

$R_1$ eine Hydroxy- oder Acetoxy-Gruppe und

$R_3$ die Acetalreste

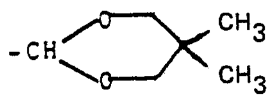 , 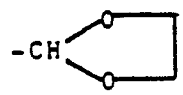 oder 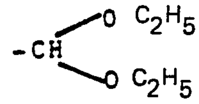

bedeuten.

Die Verbindungen der allgemeinen Formel I stellen sowohl (5E)- als auch (5Z)- Isomere dar.

Die Hydroxygruppen $R_1$ und in W können funktionell abgewandelt sein, beispielsweise durch Veresterung, wobei die freie oder abgewandelte Hydroxygruppe in W α- oder β-ständig sein kann, wobei die freie Hydroxygruppe bevorzugt ist.

Als Acylrest kommt Acetyl in Betracht.

Als Alkylgruppe $R_2$ kommen gerad- und verzweigtkettige, Alkylreste mit 1 - 7 C-Atomen in Frage. Beispielsweise seien genannt: Methyl-, Äthyl-, Propyl-, Butyl-, Isobutyl-, tert.-Butyl-, Pentyl-, Hexyl- und Heptyl.

Als Alkylengruppe D kommen geradkettige mit 1 - 5 C-Atomen oder verzweigtkettige Alkylenreste mit 2 - 4 C-Atomen in Frage. Beispielsweise seien genannt: Methylen, Äthylen, 1,2-Propylen, Äthyläthylen, Trimethylen, Tetramethylen, Pentamethylen, 1-Methyltetramethylen und 1-Methyltrimethylen.

Die Erfindung betrifft ferner ein Verfahren zur Herstellung der erfindungsgemäßen 5-Cyanoprostacycline der allgemeinen Formel I, dadurch gekennzeichnet, daß man in an sich bekannter Weise eine Verbindung der allgemeinen Formel II

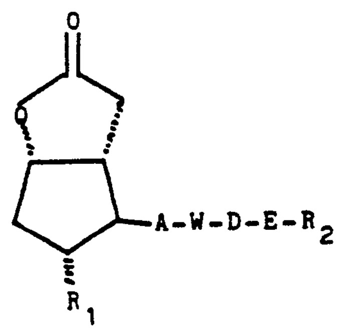

II

worin $R_1$, $R_2$, A, W, D, E die oben angegebenen Bedeutungen aufweisen, gegebenenfalls nach Schutz anwesender freier Hydroxygruppen nach Umsetzung mit einem aus dem Nitril der allgemeinen Formel III

$N\equiv C\text{-}CH_2\text{-}CH_2\text{-}CH_2\text{-}CH_2\text{-}R_3$ III

und einer Base, wie Lithiumdiisopropylamid hergestellten Carbanion, wobei $R_3$ die oben angegebene Bedeutung besitzt, gegebenenfalls nach Freisetzung geschützter Hydroxygruppen, einer Wasserabspaltung unterwirft.

Gegebenfalls kann man in den so erhaltenen Verfahrensprodukten oder in den Primärprodukten der metallorganischen Umsetzung in beliebiger Reihenfolge Isomere trennen, geschützte Hydroxygruppen freisetzen und/oder freie Hydroxygruppen verestern.

Die Umsetzung der Verbindung der allgemeinen Formel II mit der metallorganischen Verbindung, die man aus dem Nitril der allgemeinen Formel III mit Lithiumdiisoprppylamid in Äther-Tetrahydrofuran-Gemischen, gegebenenfalls in Gegenwart von Hexamethylphosphorsäuretriamid, herstellt, wird vorzugsweise bei -50° C bis -78° C, in einem für metallorganische Reaktionen geeigneten Lösungsmittel oder Lösungsmittelgemisch, vorzugsweise Diäthyläther oder Tetrahydrofuran, vorgenommen.

Die Wasserabspaltung erfolgt nach den dem Fachmann bekannten Methoden, beispielsweise durch Umsetzung der Produkte der metallorganischen Umsetzung mit einer katalytischen Menge einer Säure in einem mit Wasser nicht mischbaren Lösungsmittel, wie Toluol, Benzol, Methylenchlorid, Chloroform, Tetrachlprkohlenstoff oder Diäthyläther, vorzugsweise in Toluol oder in einem wasserbindenden Lösungsmittel, beispielsweise Essigsäureanhydrid bei Temperaturen zwischen -20° C und 100° C, vorzugsweise 0° C bis 30° C. Als Säuren kommen beispielsweise in Frage p-Toluolsulfonsäure, Schwefelsäure, Salzsäure oder Bortifluorid.

Die Einführung der Acylschutzgruppen erfolgt, indem man eine Verbindung der allgemeinen Forme III in an sich bekannter Weise mit einem Carbonsäurederivat, wie zum Beispiel Säurechlorid, Säureanhydrid u.a. in Gegenwart einer tertiären Aminbase, wie z. B. Pyridin oder Dimethylaminopyridin umsetzt.

Die Abspaltung der Silylätherschutzgruppen erfolgt beispielsweise mit Tetrabutylammoniumfluorid oder mit KF in Gegenwart eines Kronenäthers. Als Lösungsmittel sind beispielsweise geeignet Tetrahydrofuran, Diäthyläther, Dioxan oder Methylenchlorid. Die Abspaltung wird vorzugsweise bei Temperaturen zwischen 0° C und 80° C durchgeführt.

Die Verseifung der Acetylgruppe erfolgt beispielsweise mit Alkali- oder Erdalkali-carbonaten oder

-hydroxyden in einem Alkohol oder der wäßrigen Lösung eines Alkohols. Als Alkohole kommen aliphatische Alkohole in Betracht, wie zum Beispiel Methanol, Äthanol oder Butanol, vorzugsweise Methanol. Als Alkalicarbonate und -hydroxyde seien Kalium- und Natriumsalze genannt. Als Erdalkalicarbonate und -hydroxyde sind beispielsweise geeignet Calciumcarbonat, Calciumhydroxyd und Bariumcarbonat. Die setzung erfolgt bei -10°C bis 70°C, vorzugsweise bei 25°C.

Die nach dem Verfahren hergestellten Nitrile der allgemeinen Formel I stellen in Bezug auf die der Cyanogruppe benachbarten Doppelbindung Isomerengemische dar, die durch übliche Trennmethoden, wie beispielsweise Säulenchormatographie oder Schichtenromatographie getrennt werden können.

Die für das Verfahren verwendeten Nitrile der allgemeinen Formel III können beispielsweise aus 5-Bromvaleriansäureester durch Reduktion mit Diisobutylaluminiumhydrid zum 5-Bromvaleraldehyd, Acetalisierung mit einem der gewünschten Bedeutung von $R_3$ entsprechenden Alkohol oder Diol und anschließende Umsetzung mit Kaliumcyanid hergestellt werden.

Die Verbindungen dieser Erfindung wirken cytoprotektiv und bronchodilatorisch. Sie sind weiterhin geeignet zur Hemmung der Magensäuresekretion. Folglich stellen die neuen Carbacyclin-Derivate der allgemeinen Formel I wertvolle pharmazeutische Wirkstoffe der. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsepezifität der neuen Prostaglandine zeigt sich bei der Untersuchung an glattmuskelären Organen, wie zum Beispiel am Meerschwenchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie zum Beispiel Senkung des peripheren arteriellen und koronaren vaskulären Widerstandes, Inhibierung der Thrombozytenaggregation und Auflösung von Plättchenthromben, myocardiale Zytoprotektion; Senkung des systemischen Blutdruckes, ohne zugleich Schlagvolumen und koronare Durchblutung zu senken; Behandlung von Schlaganfall, Prophylaxe und Therapie koronarer Herzerkrankungen, koronarer Thrombosen, des Herzinfarktes; Behandlung Peripherer Arterienerkrankungen, Arteriosklerose und Thrombose; Prophylaxe und Therapie ischaemischer Attacken des ZNS-Systems; Therapie des Schocks; Inhibierung der Bronchokonstriktion; Inhibierung der Magensäuresekretion und Zytoprotektion der Magen- und Darmschleimhaut; Zytoprotektion in der Leber und im Pankreas; antiallergische Eigenschaften; Senkung des pulmonalen vaskulären Widerstandes und des pulmonalen Blutdruckes; Förderung der Nierendurchblutung; Anwendung anstelle von Heparin oder als Adjuvans bei der Dialyse oder Hämofiltration; Konservierung von Blutplasmakonserven, besonders von Blutplättchenkonserven; Inhibierung von Geburtswehen, Behandlung von Schwangerschaftstoxikose; Erhöhung der zerebralen Durchblutung. Außerdem besitzen die neuen Carbacyclin-Derivate antiproliferative und antidiarrhoegene Eigenschaften. Die Carbacycline dieser Erfindung können auch in Kombination, zum Beispiel mit β-Blockern, Diuretika oder Phosphodiesterasehemmern verwendet werden.

Die Einheitedosis für den pharmazeutischen akzeptablen Träger beträgt 0,01 - 100 mg.

Bei intravenöser Injektion am wachen, hypertonen ratten in Dosen von 5, 20 und 100 μg/kg Körpergewicht zeigen die erfindungsgemäßen Verbindungen eine stärkere blutdrucksenkende und länger enhaltende Wirkung als $PGE_2$ und $PGA_2$, ohne wie $PGE_2$ Durchfälle und $PGA_2$ kardiale Arrhythmien Quezulösen.

Bei intravenöser Injektion an narkotisierten Kaninchen zeigen die erfindungsgemäßen Verbindungen im Vergleich zu $PGE_2$ und $PGA_2$ eine stärkere und orheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfsund Trägerstoffe.

Die erfindungsgemäßen Wirkstoffe sollen in Verbindung mit den in der Galenik bekannten und üblichen Hilfsstoffen, zum Beispiel zur Herstellung von Blutdrucksenkern, dienen.

**Beispiel 1**

5-Cyano-2-descarboxy-2-(5,5-dimethyl-1,3-dioxan-2-yl)-16-methyl-prostacyclin

13,47 g Diisopropylamin versetzt man bei -25°C innerhalb von 15 Minuten mit 56,86 g einer 15 %-igen Lösung von Butyllithium in Hexan und rührt 1 Stunde bei - 25°C. Anschließend tropft man bei -76°C eine Lösung von 26,27 g 5-Cyano-valeraldehydneopentylacetal in 13,5 ml Tetrahydrofuran zu, rührt 20 Minuten und tropft dann eine Lösung von 15 g (1S, 5R, 6R, 7R)-6-[(E)-(3S, 4RS)-4-methyl-3-(tetrahydropyran-2-yloxy)-1-octenyl] 7-(tetrahydro-pyran-2-yloxy)-2-oxabicyclo [3.3.0]octan-3-on in 11,3 ml Tetrahydrofuran und 11,3 ml Diäthyläther zu. Man rührt 30 Minuten bei -76°C, entfernt das Kühlbad, versetzt mit gesättigter Ammoniumchloridlösung und säuert die Mischung mit 10 %-iger Zitronensäurelösung auf pH 6 an. Man extrahiert mit einer Mischung aus Äther/Hexan (1 + 1), wäscht mit Wasser, trocknet mit Magnesiumsulfat und dampft im Vakuum ein. Den Rückstand chromatographiert man mit Hexan/Äther (2 + 3) an Kieselgel. Dabei erhält man 18,05 g des

Hydroxynitrils als farbloses Öl, das man 20 Stunden mit 600 ml einer Mischung aus Essigsäure /Wasser/ Tetrahydrofuran bei 22° C rührt. Man dampft unter Zusatz von Toluol bei 30° C ein und filtriert den Rückstand mit Essigester an Kieselgel. Dabei erhält man 9,5 g des 11,15-Diols als farbloses Öl.

Zur Wasserabspaltung und Acetylierung löst man die vorstehend hergestellte Verbindung in 150 ml Toluol und 36 ml Essigsäureanhydrid, fügt 90 mg p-Toluolsulfonsäure zu und rührt 2,5 Stunden bei 20° C. Anschließend fügt man 130 ml Pyridin zu, rührt 6 Stunden bei 20° C, verdünnt mit Wasser, extrahiert mit Toluol, wäscht mit 10 %-iger Citronensäurelösung und Wasser, trocknet mit Magnesiumsulfat und dampft im Vakuum ein. Den Rückstand chromatographiert man mit Äther-Hexan-Gemischen an Kieselgel. Dabei erhält man 4,1 g des gewünschten (5E)-5-Cyano-2-descarboxy-2-(5,5-dimethyl-1,3-dioxan-2-yl)-16-methyl-prostacyclin-11,15-diacetats sowie als polarere Komponente 3,5 g des entsprechenden isomeren (5Z)-5-Cyano-2-descarboxy-2-(5,5-dimethyl-1,3-dioxan-2-yl)-16-methyl-prostacyclin-11,15-diacetats.

IR ($CHCl_3$): 2959, 2860, 2202, 1732, 1654, 1240, 972/cm

Zur Acetatabspaltung rührt man 4,1 g des vorstehend hergestellten Diacetats in 220 ml Methanol mit 5,3 g Kaliumcarbonat 16 Stunden bei 23° C. Anschließend engt man im Vakuum ein, verdünnt mit Äther, wäscht mit Sole neutral, trocknet über Magnesiumsulfat und dampft im Vakuum ein. Den Rückstand reinigt man durch Chromatographie an Kieselgel. Mit Essigester/Hexan (7 + 3) als Fließmittel erhält man 3,4 g der Titelverbindung als farbloses Öl.

IR: 3600, 3430 (breit), 2957, 2860, 2200, 1649, 970/cm

Das bei der Synthese verwendete 5-Cyano-valeraldehyd-neopentylacetal wird wie folgt hergestellt:

<u>5-Bromvaleraldehyd-neopentylacetal</u>

151,0 g 5-Bromvaleriansäuremethylester in 2500 ml Toluol werden unter Argon und Wasserausschluß bei -65 bis -70° C tropfenweise mit 840 ml DIBAH-Lösung (20 % in Toluol) versetzt und 20 Minuten gerührt. Nacheinander werden 420 ml Isopropanol und 420 ml Wasser bei -70 bis -60° C zugetropft. Man läßt auf 20° C erwärmen und rührt, bis der Niederschlag gut filtrierbar ist. Man filtriert, wäscht mit Toluol und konzentriert im Vakuum auf ca. 1000 ml.

Die erhaltene Toluol-Lösung von 5-Bromvaleraldehyd wird auf 1,28 l mit Toluol aufgefüllt, mit 120,8 g 2,2-Dimethylpropan-1,3-diol und 1,28 g p-Toluolsulfonsäure-hydrat versetzt und am Wasserabscheider unter Rückfluß erhitzt, bis die Wasserabscheidung beendet ist (1,5 Stunden). Man läßt abkühlen, gibt in 435 ml ges. $NaHCO_3$-Lösung und trennt die Phasen. Die Toluolphase wird dreimal mit je 400 ml Wasser gewaschen, mit Natriumsulfat getrocknet, im Vakuum konzentriert und dann im Ölpumpenvakuum destiliert. Man erhält 154 g vom Kp 67° C (0,7 mbar).

<u>5-Cranovaleraldehyd-neopentylacetal</u>

150,0 g 5-Bromvaleraldehyd-neopentylacetal werden in 600 ml wasserfreiem Dimethylsulfoxid mit 32,2 g Natriumcyanid unter Argon 3,5 Stunden bei 90° C gerührt. Man gießt die Reaktionslösung in 1300 ml Wasser und extrahiert dreimal mit je 500 ml Äther-Hexan 1 : 1. Die organischen Phasen werden fünfmal mit 120 ml Wasser gewaschen, mit Natriumsulfat getrocknet, im Vakuum konzentriert und im Ölpumpenvakuum destilliert. Man erhält 100,1 g vom Kp 90° C (0,6 mbar).

**Beispiel 2**

<u>(5Z)-5-Cyano-2-descarboxy-2-(5,5-dimethyl-1,3-dioxan-2-yl)-16-methyl-prostacyclin</u>

3,5 (5Z)-5-Cyano-2-descarboxy-2-(5,5-dimethyl-1,3-dioxan-2-yl)-16-methyl-prostacyclin-11,15-diacetat (hergestellt nach Beispiel 1) in 180 ml Methanol rührt man mit 4,5 g Kaliumcarbonat 16 Stunden bei 23° C. Anschließend engt man im Vakuum ein, verdünnt mit Äther, wäscht mit Sole neutral, trocknet über Magnesiumsulfat und dampft im Vakuum ein. Den Rückytand reinigt man durch Chromatographie an Kieselgel. Mit Essigester/ Hexan (7 + 3) als Fließmittel erhält man 2,8 g der Titelverbindung als farbloses Öl.

IR: 3600, 3420 (breit), 2958, 2860, 2200, 1650, 970/cm.

**Beispiel 3**

<u>5-Cyano-2-descarboxy-16,16-dimethyl-2-(5,5-dimethyl-1,3-dioxan-2-yl)-prostacvclin</u>

In Analogie zu Beispiel 1 erhält man aus 3 g (1S, 5R, 6R, 7R)-6-[(E)-(3R)-4,4-dimethyl-3-(tetrahydropyran-2-yloxy)-1-octenyl]-7-(tetrahydropyran-2-yloxy)-2-oxabicyclo [3.3.0]octan-3-on nach chromatographischer Trennung gemäß Beispiel 1 800 mg des gewünschten (5E)-5-Cyano-2-descarboxy-16,16-dimethyl-2-(5,5-dimethyl-1,3-dioxan-2-yl)-prostacyclin-11,15-diacetats und als polarere Komponente 650 mg des entsprechenden isomeren (5Z)-5-Cyano-2-descarboxy-16,16-dimethyl-2-(5,5-dimethyl-1,3-dioxan-2-yl)-prostacyclin-11,15-diacetats.

IR: 2960, 2860, 2201, 1732, 1655, 1240, 972/cm

Nach Abspaltung der Acetatreste gemäß Beispiel 1 erhält man 620 mg der Titelverbindung als farbloses Öl.

IR: 3610, 3420 (breit), 2958, 2860, 2200, 1649, 970/cm.

**Beispiel 4**

5-Cyano-2-descarboxy-2-(5,5-dimethyl-1,3-dioxan-2-yl)-16-phenoxy-17,18,19,20-tetranor-prostacyclin

In Analogie zu Beispiel 1 erhält man aus 4 g (1S, 5R, 6R, 7R)-6-[(E)-(3R)-4-phenoxy-3-(tetrahydropyran-2-yloxy)-1-butenyl]-7-(tetrahydropyran-2-yloxy)-2-oxabicyclo[3.3.0]octan-3-on nach chromatographischer Trennung (gemäß Beispiel 1) 995 mg des gewünschten (5E)-5-Cyano-2-descarboxy-2-(5,5-dimethyl-1,3-dioxan-2-yl)-16-phenoxy-17,18,19,20-tetranor-prostacyclin-11,15-diacetats und als polarere Komponente 780 mg des entsprechenden isomeren (5Z)-5-Cyano-2-descarboxy-2-(5,5-dimethyl-1,3-dioxan-2-yl)-16-phenoxy-17,16,19,20-tetranor-prostacyclin-11,15-diacetats.

IR: 2958, 2850, 2205, 1735, 1658, 1601, 1588, 1245, 976/cm.

Nach Abspaltung der Acetatreste aus dem E-Isomeren gemäß Beispiel 1 erhält man 710 mg der Titelverbindung als farbloses Öl.

IR: 3610, 3405 (br.), 2958, 2851, 2203, 1651, 1601, 1588, 974/cm

**Beispiel 5**

5-Cyano-2-descarboxy-2-(5,5-dimethyl-1,3-dioxan-2-yl)-(15RS)-15-methyl-prostacyclin

In Analogie zu Beispiel 1 erhält man aus 2 g (1S, 5R, 6R, 7R)-6-[(E)-(3RS)-3-methyl-3-(tetrahydropyran-2-yloxy-1-octenyl]-7-(tetrahydropyran-2-yloxy)-2-oxabicyclo[3.3.0]octan-3-on nach chromatographischer Trennung gemäß Beispiel 1 530 mg des gewünschten (5E)-5-Cyano-2-descarboxy-(5,5-dimethyl-1,3-dioxan-2-yl)-(15RS)-15-methyl-prostacyclin-11,15-diacetats und als polarere Komponente 432 mg des entsprechenden isomeren (5Z)-5-Cyano-2-descarboxy-2-(5,5-dimethyl-1,3-dioxan-2-yl)-(15RS)-15-methyl-prostacyclin-11,15-diacetats.

IR: 2955, 2858, 2200, 1738, 1652, 1245, 976/cm.

Nach Abspaltung der Acetatreste aus dem E-Isomeren gemäß Beispiel 1 erhält man 395 mg der Titelverbindung als farbloses Öl.

IR: 3605, 3410 (br.), 2957, 2860, 2201, 1651, 974/cm

**Beispiel 6**

5-Cyano-2-descarboxy-2-(5,5-dimethyl-1,3-dioxan-2-yl)-(16RS)-16-methyl-16,18,19,19-tetradehydro-prostacyclin

In Analogie zu Beispiel 1 erhält man aus 2,85 g (1S, 5R, 6R, 7R)-6-[(E)-(3S,4RS)-4-methyl-3-(tetrahydropyran-2-yloxy)-1-octen-6-inyl]-7-(tetrahydropyran-2-yloxy)-2-oxabicyclo[3.3.0] octan-3-on nach chromatographischer Trennung gemäß Beispiel 1 760 mg des gewünschten (5E)-5-Cyano-2-descarboxy-2-(5,5-dimethyl-1,3-dioxan-2-yl)-(16RS)-16-methyl-18,18,19,19-tetradehydro-prostacyclin-11,15-diacetats und als polarere Komponente 625 mg des entsprechenden isomeren (5Z)-5-Cyano-2-descarboxy-2-(5,5-dimethyl-1,3-dioxan-2-yl)-(16RS)-16-methyl-18,18,19,19-tetradehydro-prostacyclin-11,15 diacetats.

IR: 2962, 2858, 2206, 1735, 1651, 1248, 976/cm

Nach Abspaltung der Acetatreste aus dem E-Isomeren gemäß Beispiel 1 erhält man 585 mg der Titelverbindung als farbloses Öl.

IR: 3604, 3410 (br.), 2959, 2857, 2204, 1652, 974/cm

**Beispiel 7**

5-Cyano-2-descarboxy-2-(5,5-dimethyl-1,3-dioxan-2-yl)-(16RS)-16,20-dimethyl-18,18,19,19-tetradehydro-prostacyclin

In Analogie zu Beispiel 1 erhält man aus 3 g (1S, 5R, 6R, 7R), 6-[(E)-(3S,4RS)-4-methyl-3-(tetrahydropyran-2-yloxy)-1-nonen-6-inyl]-7-(tetrahydropyran-2-yloxy)-2-oxabicyclo[3.3.0]octan-3-on nach chromatographischer Trennung gemäß Beispiel 1 775 mg des gewünschten (5E)-5-Cyano-2-descarboxy-2-(5,5-dimethyl-1,3-dioxan-2-yl)-(16RS)-16,20-dimethyl-18,18,19,19-tetradehydro-prostacyclin-11,15-diacetats und als polarere Komponente 630 mg des entsprechenden isomeren (5Z)-5-Cyano-2-descarboxy-2-(5,5-dimethyl-1,3-dioxan-2-yl)-(16RS)-16,20-dimethyl-18,18,19,19-tetradehydro-prostacyclin-11,15-diacetats.

IR: 2960, 2862, 2202, 1735, 1656, 1248, 974/cm

Nach Abspaltung der Acetatreste aus dem E-Isomeren gemäß Beispiel 1 erhält man 590 mg der

Titelverbindung als farbloses Öl.

IR: 3600, 3420 (br.), 2960, 2858, 2202, 1655, 974/cm

**Beispiel 8**

5-Cyano-2-descarboxy-2-(1,3-dioxolan-2-yl)-(16RS)-16-methyl-prostacyclin

13,47 g Diisopropylamin versetzt man bei -25°C innerhalb von 15 Minuten mit 56,86 g einer 15 %-igen Lösung von Butyllithium in Hexan. Nach 1 Stunde tropft man bei -76°C eine Lösung von 20,67 g 5-Cyano-valeraldehyd-diäthylenacetal in 12 ml Tetrahydrofuran zu, rührt 20 Minuten und tropft dann eine Lösung von 6,26 g (1S, 5R, 6R, 7R)-6-[(E)-(3S, 4RS)-3-hydroxy-4-methyl-1-octenyl]-7-hydroxy-2-oxabicyclo[3.3.0]octan-3-on in 12 ml Tetrahydrofuran und 12 ml Diäthyläther zu. Man rührt 30 Minuten bei -76°C, entfernt das Kühlbad, versetzt mit gesättigter Ammoniumchloridlösung und säuert die Mischung mit 10 %-iger Citronensäurelösung auf pH 6 an. Man extrahiert mit einer Mischung aus Diäthyläther/Hexan (1 + 1), wäscht mit Wasser, trocknet mit Magnesiumsulfat und dampft im Vakuum ein. Den Rückstand chromatographiert man mit Hexan/Diäthyläther (1/9) an Kieselgel. Man erhält 7 g des Hydroxynitrils als farbloses Öl, das man zur Wasserabspaltung in 120 ml Toluol löst, mit 70 mg p-Toluolsulfonsäure versetzt und 2 Stunden bei 20°C rührt. Anschließend wäscht man mit Natriumhydrogencarbonat und Wasser, trocknet über Magnesiumsulfat und dampft im Vakuum ein. Den Rückstand chromatographiert man an Kieselgel mit Hexan und steigendem Essigesteranteil. Dabei erhält man als polarere Komponente 2,30 g (5Z)-5-Cyano-2-descarboxy-2-(1,3-dioxolan-2-yl)-(16RS)-16-methyl-prostacyclin und 3,01 g der Titelverbindung (5E-Isomeres) als farbloses Öl.

IR: 3600, 3420 (br.), 2955, 2658, 2207, 1653, 974, 946/cm

Das zur Synthese verwendete 5-Cyano-valeraldehyd-diäthylenacetal wird wie folgt hergestellt:

8a) 5-Brom-valeraldehyd:

Zu einer Lösung von 46,75g Brom-valeriansäuremethylester in 2,5 l Toluol tropft man bei -70°C unter Rühren 271 ml einer 1,2 M-Lösung von Diisobutylaluminiumhydrid in Toluol, rührt 30 Minuten nach und tropft nacheinander 50 ml Isopropylalkohol und 135 ml Wasser zu, rührt 2 Stunden bei Raumtemperatur, filtriert vom Niederschlag und dampft im Vakuum ein. Man erhält 43 g 5-Brom-valeraldehyd, die ohne Reinigung weiter umgesetzt werden.

8b) 5-Brom-valeraldehyd-diäthylenacetal:

43 g 5-Brom-valeraldehyd, 51 ml Äthylenglykol 500 mg p-Toluolsulfonsäure und 1 l Toluol werden 6 Stunden unter Rückfluß zur Wasserabscheider erhitzt. Nach dem Abkühlen wird mit Natriumhydrogencarbonatlösung und Sole geschüttelt, unter Magnesiumsulfat getrocknet und das Toluol im Vakuum abdestilliert. Der flüssige Rückstand wird bei 0,2 mbar destilliert. Man erhält 25,06 g vom Kp 64°C.

8c) 5-Cyano-valeraldehyd-diäthylenacetal:

32,7 g 5-Brom-valeraldehyd-diäthylenacetal werden in 140 ml Dimethylsulfoxid mit 16,24 g Natriumcyanid 6 Stunden unter Argon bei 90°C gerührt. Man kühlt ab, verdünnt mit 300 ml Wasser, extrahiert mehrmals mit Diäthyläther/Hexan (1 : 1), wäscht den Extrakt mit Sole, trocknet über Magnesiumsulfat und dampft im Vakuum das Lösungsmittel ab. Den flüssigen Rückstand destilliert man bei 0,8 mbar. Man erhält 16,6 g vom Kp 87°C.

**Beispiel 9**

5-Cyano-2-descarboxy-2-(1,3-dioxolan-2-yl)-16,16-dimethyl-prostacyclin

In Analogie zu Beispiel 6 erhält man aus 700 mg (1S, 5R, 6R, 7R)-6-[(E)-(3S)-4,4-dimethyl-3-(tetrahydropyran-2-yloxy)-1-octenyl]-7-(tetrahydropyran-2-yloxy)-2-oxabicyclo[3.3.0]octan-3-on 310 mg der Titelverbindung als farbloses Öl.

IR: 3600, 3410 (br.), 2560, 2661, 2203, 1649, 975, 948/cm

**Beispiel 10**

5-Cyano-2-descarboxy-2-diäthoxymethyl-(16RS)-16-methyl-prostacyclin

1,347 g Diisopropylamin versetzt man bei -25°C innerhalb von 15 Minuten mit 5,686 g einer 15 %-igen Lösung von Butyllithium in Hexan. Nach 1 Stunde tropft man bei -76°C eine Lösung von 2,47 g 5-Cyano-valeraldehyddiäthylacetal in 15 ml Tetrahydrofuran zu, rührt 20 Minuten und tropft dann eine Lösung von 630 mg (1S, 5R, 6R, 7R)-6-[(E)-(3S, 4RS)-3-hydroxy-4-methyl-1-octenyl]-7-hydroxy-2-oxabicyclo[3.3.0]octan-3-on in 3 ml Tetrahydrofuran und 3 ml Diäthyläther zu. Man rührt 30 Minuten bei -76°C, entfernt das Kühlbad, versetzt mit gesättigter Ammoniumchloridlösung und extrahiert mit Diäthyläther/Hexan (1 : 1), wäscht mit Sole, trocknet über Magnesiumsulfat und dampft im Vakuum ein. Den Rückstand chromatographiert man an Kieselgel mit Hexan/Diäthyläther (1/9) und erhält 720 mg des Hydroxynitrils als farbloses Öl, das man zur Wasserabspaltung in 15 ml Toluol löst und mit 10 mg p-Toluolsulfonsäure 2 Stunden bei 20°C rührt. Anschließend verdünnt man

mit Diäthyläther, schüttelt mit Natriumhydrogencarbonatlösung und Wasser, trocknet über Magnesiumsulfat und dampft im Vakuum ein. Den Rückstand chromatographiert man an Kieselgel mit Hexan/Essigester (1/4) und erhält als polarere Komponente 200 mg (5Z)-5-Cyano-2-descarboxy-2-diäthoxymethyl-(16RS)-16-methyl-prostacyclin und 305 mg der Titelverbindung (5E-Isomeres) als farbloses Öl.

IR: 3605, 3410 (br.), 2959, 2860, 2201, 1650, 976/cm

Das zur Synthese verwendete 5-Cyanovaleraldehyd-diäthylacetal erhält man in Analogie zu Beispiel 8c) aus 5-Bromvaleraldehyd-diäthylacetal [Bull. Chem. Soc. Jp. 49, 1989 (1976)] durch Umsetzung mit Natriumcyanid: Kp. 82° C bei 0,7 mbar.

**Beispiel 11**

5-Cyano-2-descarboxy-2-(5,5-dimethyl-1,3-dioxan-2-yl)-(16S)-16-methyl-prostacyclin

Man versetzt 8,76 g Diisopropylamin bei -25° C mit 73 ml einer 1,2 M Lösung von Butyllithium in Hexan. Nach 1 Stunde tropft man bei -78° C eine Lösung von 17,08 g 5-Cyano-valeraldehydneapentylacetal in 8 ml Tetrahydrofuran zu. Nach 20 Minuten tropft man eine Lösung von 9,75 g (1S, 5R, 6R,7R)-6-[(E)-(3S, 4S)-4-methyl-3-(tetrahydropyran-2-yloxy)-1-octenyl]-7-(tetrahydropyran-2-yloxy)-2-oxabicyclo[3.3.0]octan-3-on (s.J. Org. Chem. 38, 1250 [1979]) in 8 ml Tetrahydrofuran und 8 ml Diäthyläther zu. Nach weiteren 30 Minuten bei -78° C versetzt man mit gesättigter Ammoniumchloridlösung und säuert mit Citronensäure auf pH 6 an. Man extrahiert mit Äther/Hexan (1 + 1), wäscht mit Wasser, trocknet über Magnesiumsulfat und dampft im Vakuum ein. Den Rückstand chromatographiert man mit Hexan/Äther (2 + 3) an Kieselgel und erhält 12 g des Hydroxynitrils als Öl. Zur Abspaltung der Tetrahydropyranylätherschutzgruppen rührt man 24 Stunden mit 400 ml einer Mischung aus Essigsäure/Wasser/Tetrahydrofuran (65/ 35/10), dampft im Vakuum ein und filtriert den Rückstand mit Hexan/Essigester (2 + 8) über Kieselgel. Man erhält 6 g des 11,15-Diols als farbloses Öl.

Zur Wasserabspaltung und Acetylierung löst man das Öl in 100 ml Toluol und 23 ml Essigsäureanhydrid, fügt 60 mg P-Toluolsulfonsäure zu und rührt 3 Stunden bei 20°C. Danach versetzt man mit 85 ml Pyridin. Nach weiteren 6 Stunden verdünnt man mit Wasser, extrahiert mit Toluol, wäscht mit 10 %-iger Citronensäurelösung und Wasser, trocknet mit Magnesiumsulfat und dampft im Vakum ein. Der Rückstand wird an Kieselgel mit Äther/Hexan (1 + 1) chromatographiert. Man erhält 2,60 g des gewünschten (5E)-5-Cyano-2-descarboxy-2-(5,5-dimethyl-1,9-dioxan-2-yl)-(16S)-16-methyl-prostacyclin-11,15-diacetats sowie als polarere Komponente 2,20 g des entsprechenden isomeren (5Z)-5-Cyano-2-descarboxy-2-(5,5-dimethyl-1,3-dioxan-2-yl)-(16S)-16-methyl-prostacyclin-11,15-diacetats.

IR (5E-Isomeren): 2960, 2861, 2202, 1735, 1655, 976/cm.

Zur Acetatabspaltung rührt man 2,60 g des vorstehend hergestellten (5E)-11,15-Diacetats in 140 ml Methanol mit 3,45 g Kaliumcarbonat 16 Stunden bei 20° C. Zur Aufarbeitung engt man im Vakuum ein, verdünnt mit Äther, wäscht mit Sole, trocknet über Magnesiumsulfat und dampft im Vakuum ein. Den Rückstand reinigt man durch Chromatographie an Kieselgel mit Hexan/Essigester (3 + 7) und erhält 2,19 g der Titelverbindung als farbloses Öl.

IR: 3600, 3420 (breit), 2958, 2861, 2203, 1651, 974/cm.

**Beispiel 12**

5-Cyano-2-descarboxy-2-(5,5-dimethyl-1,3-dioxan-2-yl)-(16R)-16-methyl-prostacyclin

In Analogie zu Beispiel 11, aber unter Verwendung des anderen 16-Epimeren, erhält man aus 5 g (1S, 5R, 6R, 7R)-6-[(E)-(3S, 4R)-4-methyl-3-(tetrahydropyran-2-yloxy)-1-octenyl]-7-(tetrahydropyran-2-yloxy)-2-oxabicyclo[3.3.0]octan-3-on (J. Org. Chem. 38, 1250 [1973]) 1,15 g der Titelverbindung als Öl.

IR: 3600, 3420 (breit), 2953, 2861, 2202, 1650, 974/cm.

**Patentansprüche**

1. 5-Cyanopostacycline der allgemeinen Formel I

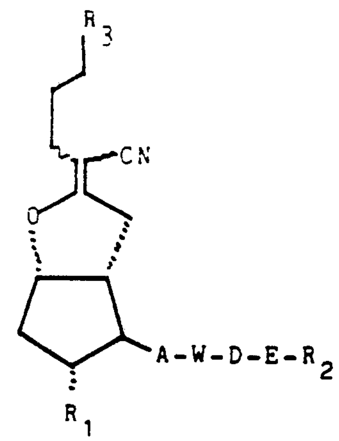

I

worin

A eine trans-$CH{=}CH$- oder -$C{\equiv}C$-Gruppe,

W eine Hydroxymethylen-, Acetoxymethylen-,

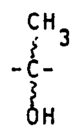

oder

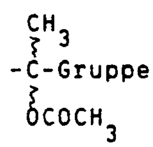

-Gruppe,

wobei die OH- oder Acetoxy-Gruppe α- oder β-ständig sein kann,

D und E gemeinsam eine direkte Bindung oder

D eine geradkettige Alkylengruppe mit 1 bis 5 C-Atomen oder eine verzweigte Alkylengruppe mit 2 bis 5 C-Atomen,

E ein Sauerstoffatom, eine -$C{\equiv}C$-Bindung oder eine direkte Bindung,

$R_2$ eine $C_1$-$C_7$-Alkyl-Gruppe oder Phenyl

$R_1$ eine Hydroxy- oder Acetoxy-Gruppe und

$R_3$ die Acetalreste

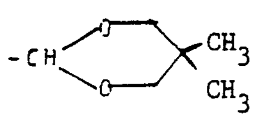

,

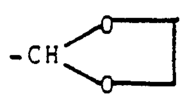

oder

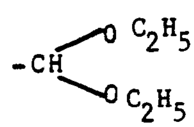

bedeuten.

2. Verfahren zur Herstellung von 5-Cyanoprostacyclinen der allgemeinen Formel I, dadurch gekennzeichnet, daß man in an sich bekannter Weise eine Verbindung der allgemeinen Formel II

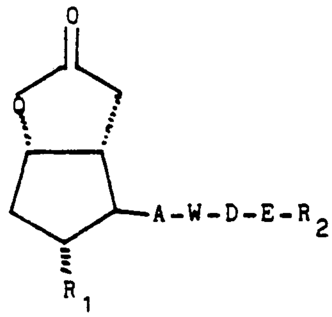

II

worin $R_1$, $R_2$, A, W, D, E die oben angegebenen Bedeutungen aufweisen, gegebenenfalls nach Schutz anwesender freier Hydroxygruppen nach Umsetzung mit einem aus dem Nitril der allgemeinen Formel III

$N \equiv C\text{-}CH_2\text{-}CH_2\text{-}CH_2\text{-}CH_2\text{-}R_3$ III

und Lithiumdiisopropylamid hergestellten Carbanion, wobei $R_3$ die oben angegebene Bedeutung besitzt, gegebenenfalls nach Freisetzung geschützter Hydroxygruppen, einer Wasserabspaltung unterwirft.

3. 5-Cyano-2-descarboxy-2-(5,5-dimethyl-1,3-dioxan-2-yl)-16-methyl-prostacyclin.

4. Arzneimittel, bestehend aus einer oder mehreren verbindungen des Anspruches 1 und üblichen Hilfs- und Trägerstoffen.

## Claims

1. 5-Cyanoprostacyclines of the general formula I

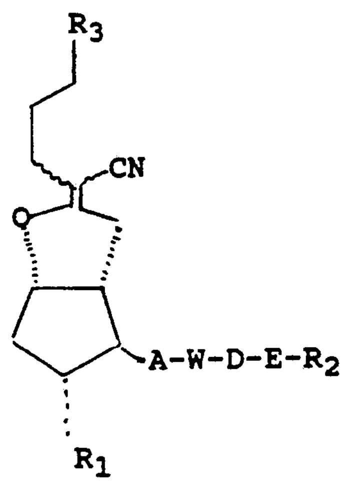

I

wherein

A represents a trans-$CH=CH$- or -$C\equiv C$- group,

W represents a hydroxymethylene, acetoxymethylene,

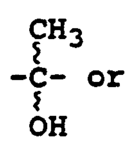 or 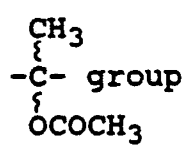 group,

wherein the OH or acetoxy group may have the α- or β-configuration,

D and E together represent a direct bond or

D represents a straight-chained alkylene group having from 1 to 5 C atoms or a branched alkylene group having from 2 to 5 C atoms,

E represents an oxygen atom, a -C≡C- bond or a direct bond,

$R_2$ represents a $C_1$-$C_7$-alkyl group or phenyl,

$R_1$ represents a hydroxy or acetoxy group and

$R_3$ represents the acetal radicals

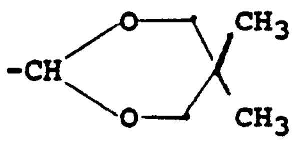 , 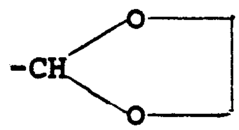 or 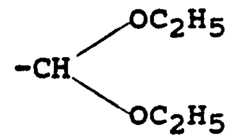 .

2. Process for the preparation of 5-cyanoprostacyclines of the general formula I, characterised in that, in a manner known per se, a compound of the general formula II

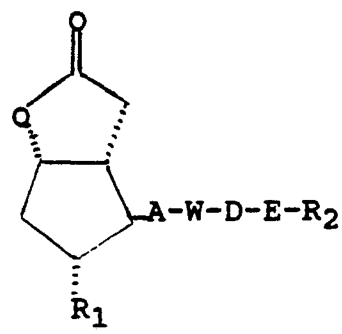

II

wherein $R_1$, $R_2$, A, W, D and E have the meanings given above, optionally after protecting free hydroxy groups present and after reaction with a carbanion produced from the nitrile of the general formula III

$N \equiv C\text{-}CH_2\text{-}CH_2\text{-}CH_2\text{-}CH_2\text{-}R_3$ III

and from lithium diisopropylamide, wherein $R_3$ has the meaning given above, is subjected, optionally after freeing protected hydroxy groups, to splitting off of water.

3. 5-cyano-2-decarboxy-2-(5,5-dimethyl-1,3-dioxan-2-yl)-16-methylprostacycline.

4. Medicament, comprising one or more compounds of claim 1 and customary adjuvants and carriers.

## Revendications

1. Cyano-5 prostacyclines répondant à la formule générale I:

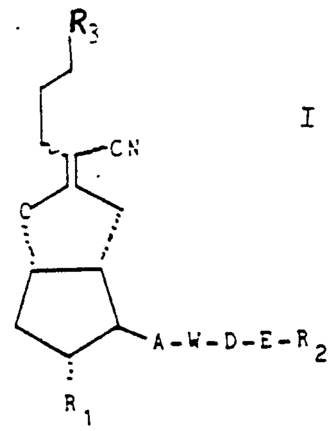

I

dans laquelle

A représente un radical $-CH=CH-$ trans ou un radical $-C\equiv C-$,

W représente un radical hydroxyméthylène, acétoxyméthylène,

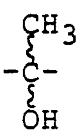 ou 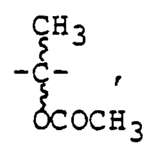,

les radicaux -OH et acétoxy pouvant avoir la configuration α ou la configuration β,

D et E forment ensemble une liaison directe, ou

D représente un radical alkylène linéaire qui contient de 1 à 5 atomes de carbone, ou un radical alkylène ramifié qui contient de 2 à 5 atomes de carbone, et

E représente un atome d'oxygène, une liaison $-C\equiv C-$ ou une liaison directe,

$R_2$ représente un alkyle en $C_1$-$C_7$ ou un phényle,

$R_1$ représente un radical hydroxy ou acétoxy et

$R_3$ représente un radical d'acétal répondant à l'une des formules suivantes :

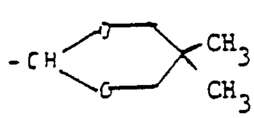, 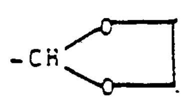 et 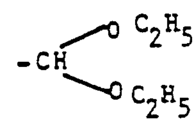

2. Procédé pour préparer des cyano-5 prostacyclines de formule générale I, procédé caractérisé en ce que, en opérant de manière connue, on fait d'abord réagir un composé répondant à la formule générale II:

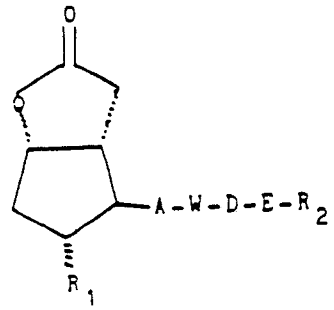

II

dans laquelle $R_1$, $R_2$, A, W, D et E ont les significations qui leur ont été données ci-dessus, éventuellement après en avoir protégé des radicaux hydroxy libres présents, avec un carbanion préparé à partir du nitrile répondant à la formule générale III:

$N \equiv C\text{-}CH_2\text{-}CH_2\text{-}CH_2\text{-}CH_2\text{-}R_3$ III

dans laquelle $R_3$ a la signification qui lui a été donnée plus haut, et avec le di-isopropylamidure de lithium, puis on soumet le produit formé, éventuellement après en avoir libéré des radicaux hydroxy protégés, à une réaction d'enlèvement d'eau.

3. Cyano-5 décarboxy-2 (diméthyl-5,5 dioxanne-1,3 yl-2)-2 méthyl-16 prostacycline.

4. Médicament constitué d'un ou de plusieurs composés selon la revendication 1 et d'adjuvants et excipients usuels.